**Europäisches Patentamt**

**European Patent Office**

**Office européen des brevets**

(19)

(11) Veröffentlichungsnummer: **0 001 796**
**B1**

(12) **EUROPÄISCHE PATENTSCHRIFT**

(45) Veröffentlichungstag der Patentschrift: **10.06.81**

(21) Anmeldenummer: **78101204.2**

(22) Anmeldetag: **24.10.78**

(51) Int. Cl.³: **A 61 K 31/49,**
**A 61 K 31/47,**
**A 61 K 31/505,**
**A 61 K 31/53,**
**A 61 K 31/635,**
**A 61 K 31/435 //(A61K31/49,**
**31/435), (A61K31/47,**
**31/435), (A61K31/505,**
**31/435), (A61K31/53,**
**31/435), (A61K31/635,**
**31/435), (A61K31/435,**
**31/135)**

(54) **Mittel gegen Malaria.**

(30) Priorität: **28.10.77 DE 2748333**

(43) Veröffentlichungstag der Anmeldung:
**16.05.79 Patentblatt 79/10**

(45) Bekanntmachung des Hinweises auf die Patenterteilung:
**10.06.81 Patentblatt 81/23**

(84) Benannte Vertragsstaaten:
**BE CH DE FR GB NL**

(56) Entgegenhaltungen:
**DE - A - 2 337 474**

(73) Patentinhaber: **HOECHST AKTIENGESELLSCHAFT**
**Postfach 80 03 20**
**D-6230 Frankfurt/Main 80 (DE)**

(72) Erfinder: **Raether, Wolfgang, Dr.**
**Odenwaldring 156**
**D-6072 Dreieich (DE)**
Erfinder: **Dürckheimer, Walter, Dr.**
**Im Lerchenfeld 45**
**D-6234 Hattersheim am Main (DE)**
Erfinder: **Seidenath, Hans**
**Alte Schulstrasse 9**
**D-6350 Bad Nauheim (DE)**

# Mittel gegen Malaria

Die Bekämpfung der Malaria kann entweder durch medikamentöse Prophylaxe oder Therapie des Menschen oder durch Insektizide erfolgen, die sich gegen die Überträger der Malaria (Anopheles-Mückenarten) richten.

Bei wiederholter Anwendung von Chemotherapeutica wie Chloroquine, das oft zur Massenbehandlung eingesetzt wird und relativ gut verträglich ist, hat insbesondere der am meisten pathogene Erreger der Malaria, Plasmodium falciparum, eine hohe Resistenz entwickelt. Selbst nach deutlicher Erhöhung der Dosis, die dann bereits im toxischen Bereich liegt, sind resistente Stammvarianten nicht mehr zu beeinflussen. Arzneiresistenz von Malariaerregern besteht auch gegen andere Malariamittel, wie Pyrimethamine oder andere Folsäurereduktasehemmer.

In der deutschen Offenlegungsschrift 23 37 474 werden chemotherapeutisch wirksame Tetrahydroacridon-Verbindungen beschrieben, die sich durch hohe Wirksamkeit gegen verschiedene Erreger der Malaria auszeichnen, so z.B. gegen Chloroquine-resistente Stammvarianten von Plasmodium berghei in der Maus, die auf herkömmliche Malariamittel wie 4-Aminochinolinderivate nicht in ausreichendem Maße ansprechen.

Es wurde nun ein Mittel gegen Malaria gefunden, das gekennzeichnet ist durch einen Gehalt an 7-Chlor-10-hydroxy-3-(4-trifluormethylphenyl)-3,4-dihydroacridin-1,9-(2H,10H)-dion (Floxacrin) oder einem seiner Salze mit einer physiologisch verträglichen Säure oder Base, in Mischung mit

a) 6-Methoxy-$\alpha$-(5-vinyl-2-chinuclidinyl)-4-chinolinmethanol (Chinin),

b) 7-Chlor-4-(diäthylamino-1-methyl-butylamino)-chinolin (Chloroquine),

c) $\alpha$-(2-Piperidyl)-2,8-bis(trifluormethyl)-4-chinolinmethanol (Mefloquine),

d) 8-(4-Amino-1-methylbutylamino)-6-methoxy-chinolin (Primaquine),

e) 2,4-Diamino-5-p-chlorphenyl-6-äthylpyrimidin (Pyrimethamine),

f) 4,6-Diamino-1-(p-chlorphenyl)-1,2-dihydro-2,2-dimethyl-s-triazin (Cycloguanil),

g) 2,4-Diamino-5-(3,4,5-trimethoxybenzyl)-pyrimidin (Trimethoprim),

h) N'-(5,6-Dimethoxy-4-pyrimidyl)-sulfanilamid (Sulfadoxine) oder

i) 4,4'-Diaminodiphenylsulfon (Dapsone),

oder mit einem Salz der Verbindungen a) bis i) mit einer verträglichen Säure oder Base, in einem Gewichtsverhältnis zwischen 25:1 und 1:300 (Floxacrin zu a) bis i)), mit einem pharmazeutisch üblichen Träger und/oder Konstituents.

Je nach Art der neben dem Tetrahydroacridon verwendeten Mischungskomponente variierten die zweckmäßig zu verwendenden Mengenverhältnisse. Bei Verwendung von Floxacrine in Mischung mit Chinin hat ein Verhältnis von 1:10 bis 1:160, insbesondere 1:40 bis 1:80; in Mischung mit Chloroquine von 4:1 bis 1:20, insbesondere 2:1 bis 1:8; in Mischung mit Mefloquine von 1:1,25 bis 1:10, insbesondere 1:1,25 bis 1:5; in Mischung mit Primaquine von 1:1,25 bis 1:20, insbesondere 1:2,5 bis 1:20; in Mischung mit Pyrimethamine von 21:1 bis 1:1,5, insbesondere 10,4:1 bis 1:1,5; in Mischung mit Cycloguanil von 1:35 bis 1:284, insbesondere 1:142 bis 1:284; in Mischung mit Trimethroprim von 1:10 bis 1:80, insbesondere 1:40 bis 1:80; in Mischung mit Sulfadoxine von 1:1 bis 1:32, insbesondere 1:1 bis 1:6, in Mischung mit Dapsone von 1:5 bis 1:80, insbesondere 1:10 bis 1:80 die beste überadditive Wirkung gezeigt.

Die zu verabreichenden Mengen variieren entsprechend der individuellen Wirksamkeit der verwendeten Komponenten. Bei der Behandlung der Nagermalaria (P. berghei) ergeben z.B. Mischungen von Floxacrine mit Chinin, Cycloguanil, Trimethoprim oder Dapsone günstige Effekte, wenn sie in einer Gesamtdosis pro Verabreichung von 7,5—178 mg/kg, vorzugsweise 26,25—102,5 mg/kg oral angewendet werden. Dem gegenüber sind Mischungen von Floxacrine mit Chloroquine, Mefloquine, Primaquine, Pyrimethamine oder Sulfadoxine bereits in Gesamtdosen zwischen 1,37 und 21,25 mg/kg pro Verabreichung gut wirksam, vorzugsweise zwischen 1,56 und 11,25 mg/kg.

Der Vorteil einer Behandlung von Patienten, die an Malaria leiden, mit einer Wirkstoffmischung gegenüber einer Behandlung mit einem einzelnen Wirkstoff liegt in der Wirkung der Mischung auf resistente Erreger, in einer reduzierten Gesamtdosis mit verminderter Toxizität und in einer geringeren Möglichkeit des Erregers zur Resistenzentwicklung.

Die neuen Mittel gemäß der Erfindung sind bereits in wesentlich geringeren Dosierungen malarizid wirksam als die Einzelkomponenten und bewirken bei Versuchstieren längere Überlebenszeiten als diese. Arzneiresistente P. berghei-Stämme, z.B. eine Chloroquine-resistente Stammvariante von P. berghei, werden insbesondere durch die Kombination Floxacrine mit Chloroquine sehr günstig im Sinne einer überadditiven Wirkung beeinflußt. Die Verträglichkeit der neuen Mittel ist gut.

Die neuen Mittel gemäß der Erfindung können oral oder parenteral in Dosen von 5 bis 50 mg/kg Körpergewicht verabreicht werden. Dabei werden als Mittel gegen Malaria Dosierungseinheiten wie Dragées oder Kapseln zur oralen Applikation bzw. Ampullen zur Injektion mit jeweils 50 bis 200 mg Wirkstoff bevorzugt. Solche Dosierungseinheiten werden im allgemeinen ein- bis dreimal täglich je nach Kondition des Patienten appliziert.

Zur oralen Applikation kommen insbesondere Tabletten, Dragées, Kapseln, Pulver oder Granulate in Betracht, welche das Gemisch der Wirkstoffe zusammen mit üblichen Hilfs- und Trägerstoffen wie Stärke, Cellulosepulver, Talcum, Magnesiumstearat, Zucker, Gelatine, Calciumcarbonat, feinverteilter Kieselsäure, Carboxymethylcellulose oder ähnlichen Stoffen enthalten.

Zur parenteralen Applikation, insbesondere zur intramuskulären Injektion, kommen sterile Suspensionen in Betracht wie ölige Suspensionen, die unter Verwendung von fetten Ölen wie Olivenöl, Sesamöl, Ricinusöl oder synthetischen Triglyceriden, ggf. unter Verwendung von grenzflächenaktiven Stoffen wie Sorbitanfettsäureester, hergestellt werden. Daneben kommen wäßrige Suspensionen in Betracht, die z.B. unter Verwendung von äthoxylierten Sorbitan-Fettsäureestern, ggf. unter Zusatz von Verdickungsmitteln wie Polyäthylenglykol oder Carboxymethylcellulose, hergestellt werden.

Zur Erprobung der Wirkung werden die Mittel gemäß der Erfindung Mäusen in wäßrigen Suspensionen mit Hilfe einer Schlundsonde verabreicht. Zu diesem Zweck werden sie in Wasser oder einem anderen geeigneten Lösungsmittel gelöst, oder sie werden in 2%igem wäßrigem Tylose-Schleim suspendiert; dabei wird 100 mg der Testsubstanz in 1 ml des wäßrigen Tylose-Schleimes eingebracht; durch Zugabe von Wasser wird die gewünschte Wirkstoffkonzentration hergestellt. Als günstige Applikationsmenge pro Maus erweist sich 0,5 ml pro 20 g Körpergewicht. Vor der Verabreichung der Mittel wird eine gleichmäßige Verteilung der Wirkstoffe in der Suspension durch Ultrabeschallung erreicht.

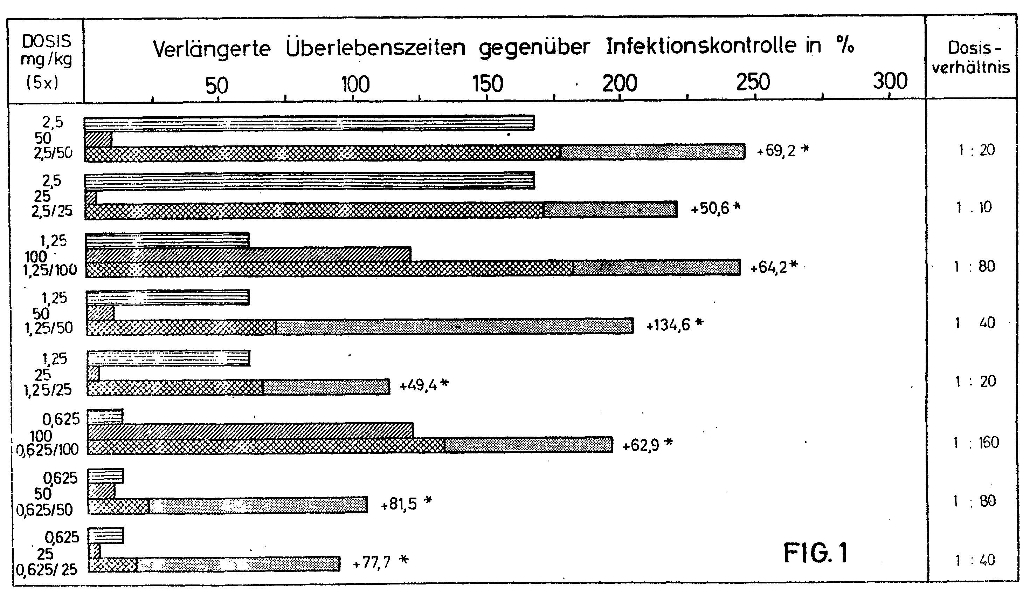

Im folgenden Versuchsbericht wird die Überlegenheit der erfindungsgemäßen Mittel gegenüber der alleinigen Anwendung jeweils eines Wirkstoffes dargestellt. Als Parameter für den überadditiven (synergistischen) Effekt, der bei bestimmten Mengenverhältnissen der Kombinationen von Floxacrine mit den genannten Marariamitteln auftritt, wird die verlängerte Überlebenszeit in Prozent herangezogen, die nach Abzug der summierten prozentualen Überlebenszeiten nach alleiniger Anwendung eines Wirkstoffes von der Überlebenszeit nach Anwendung einer Wirkstoffmischung resultiert. Zur Ermittlung der in den Abbildungen 1—9 zeichnerisch dargestellten prozentualen verlägerten Überlebenszeiten werden die in den Tabellen 1—9 aufgeführten Ergebnisse der verlängerten Überlebenszeiten in Tagen post infectionem der behandelten Gruppen nach Abzug der Überlebenszeiten der unbehandelten und infizierten Kontrollen verwendet (vgl. Tabellen 1—9, letzte Spalte).

Für die zeichnerische Darstellung der prozentualen Überlebenszeiten (ÜZ) in den Abbildungen 1—9 gelten folgende Symbole:

ÜZ: FLOXACRINE (1)

ÜZ: KOMBINATIONSPARTNER (2)

ÜZ nach Kombination von 1 und 2

Anteil additiver Effekt [ÜZ(1)+ÜZ(2)]

Anteil überadditiver (synergistischer) Effekt

= verlängerte Überlebenszeit

Die Versuche wurden an einem arzneiempfindlichen Plasmodium berghei-Stamm (K 173, Vincke und Lips, 1048) und einer Chloroquine-resistenten Linie dieses Stammes durchgeführt. NMRI-Mäuse (18—20 g Körpergewicht) männlichen und weiblichen Geschlechtes wurden in Makrolon[R]-Käfigen Typ I bei konstanter Raumtemperatuur (21°C) und einer relativen Luftfeuchtigkeit von 65% gehalten. Als Futter erhielten sie Altromin[R] und Wasser ad libitum. Jede Maus erhielt 6 Millionen mit ungeschlechtlichen Formen von P. berghei parasitierte Erythrozyten, die nach vorheriger Auszählung in einer Zählkammer und Suspendierung in physiologischer NaCl-Lösung auf die entsprechende Dichte eingestellt worden waren, intraperitoneal appliziert. Zur Kontrolle einer angegangenen Infektion und des Parasitämieverlaufes wurden beginnend an D+6 (6 Tage nach der Infektion) bis zum D+28, dreimal pro Woche Blutausstriche der behandelten und infizierten bzw. unbehandelten und infizierten Tiere angefertigt, nach Giemsa angefärbt und mikroskopisch untersucht. Die Blutentnahme erfolgte in üblicher Weise jeweils aus den Schwanzvenen. Die unbehandelten Tiere starben infolge der Infektion zwischen dem 6. und 14. Tage post infectionem. Die Schwankungsbreite der Überlebenszeiten der Infektionskontrollen ist auf eine normale biologische Variabilität des Infektionsmaterials, Tiermaterials sowie eine üblicherweise auftretende Fehlerbreite der Auszähltechnik der parasitierten Erythrozyten zurückzuführen. Zweimal täglich vom D 0 (Tag der Infektion) bis zum D+28 wurden die behandelten und infizierten sowie die unbehandelten und infizierten Mäuse hinsichtlich der Überlebenszeit inspiziert.

Die Behandlung erfolgte oral mit Hilfe der Schlundsonde. Die erste Behandlung wurde 2 Stunden, die zweite 1 Tag; die dritte 2 Tage; die vierte 3 Tage und die fünfte 4 Tage nach der Infektion durchgeführt (+2h; D+1; D+3; D+3; D+4). Pro Dosis wurden in allen Behandlungsgruppen 10 Mäuse verwendet.

Als Maß für den malariziden Effekt der erfindungsgemäßen Mittel auf die ungeschlechtlichen Blutformen von P. berghei werden die in den Tabellen 1—9 festgestellten Überlebenszeiten (Tage post in-

0 001 796

fectionem) und die aus diesen Ergebnissen resultierenden prozentualen Überlebenszeiten herange-zogen. Es wurden solche Tiere als geheilt angesehen, die 28 Tage nach der Infektion überlebten. Sie zeigten vom D+6 bis D+28 keine Parasiten im Blutausstrich (Durchmusterung von 10,000 Erythrozyten je Blutentnahme). Am D+28 wurden die Nachbeobachtung der Tiere und die Versuche abgebrochen.

TABELLE 1: Synergistischer Effekt (überadditiver Anteil der Überlebenszeit in % — vgl. Fig. 1) nach Kombination von FLOXACRINE mit CHININSULFAT auf Blutformen von Plasmodium berghei (arzneiempfindlicher Stamm) in der NMRI-Maus

| Präparat | Dosis mg/kg (5 ×) | Dosisver-hältnis | Effekt gegen Blutformen von P. berghei, gamessen in Überlebenszeiten (ÜZ) (Tage post infectionem) beobachtete Werte | | | | | | | | | | $\bar{x}$ , s | Verlängerte ÜZ $\bar{x}$ (Präp) − $\bar{x}$ (IK) |
|---|---|---|---|---|---|---|---|---|---|---|---|---|---|---|
| Floxacrine | 2,5 | | 14 | 14 | 15 | 17 | 17 | 28 | 28 | 28 | 28 | 28 | 21,7 ± 6,7 | 13,6 |
| | 1,25 | | 9 | 10 | 12 | 12 | 13 | 14 | 14 | 14 | 16 | 16 | 13 ± 2,3 | 4,9 |
| | 0,625 | | 8 | 8 | 8 | 9 | 9 | 9 | 9 | 10 | 10 | 11 | 9,1 ± 1,1 | 1 |
| Chininsulfat | 100 | | 13 | 13 | 15 | 15 | 19 | 19 | 20 | 21 | 22 | 22 | 17,9 ± 3,6 | 9.8 |
| | 50 | | 8 | 8 | 8 | 8 | 9 | 9 | 9 | 9 | 10 | 10 | 8,8 ± 0,8 | 0,7 |
| | 25 | | 8 | 8 | 8 | 8 | 8 | 8 | 9 | 9 | 9 | 9 | 8,4 ± 0,5 | 0,3 |
| Floxacrine/ Chininsulfat | 2,5/ 50 | 1 : 20 | 28 | 28 | 28 | 28 | 28 | 28 | 28 | 28 | 28 | 28 | 28 ± 0 | 19,9 |
| | 2,5/ 25 | 1 : 10 | 17 | 20 | 28 | 28 | 28 | 28 | 28 | 28 | 28 | 28 | 26,1 ± 4,1 | 18 |
| | 1,25/100 | 1 : 80 | 28 | 28 | 28 | 28 | 28 | 28 | 28 | 28 | 28 | 28 | 28 ± 0 | 19,9 |
| | 1,25/ 50 | 1 : 40 | 19 | 19 | 21 | 23 | 25 | 27 | 28 | 28 | 28 | 28 | 24,6 ± 3,8 | 16,5 |
| | 1,25/ 25 | 1 : 20 | 16 | 16 | 16 | 16 | 17 | 17 | 18 | 18 | 19 | 20 | 17,3 ± 1,4 | 9,2 |
| | 0,625/100 | 1 : 160 | 19 | 19 | 22 | 23 | 24 | 24 | 26 | 27 | 28 | 28 | 24 ± 3,3 | 15,9 |
| | 0,625/ 50 | 1 : 80 | 10 | 11 | 12 | 12 | 16 | 16 | 19 | 20 | 24 | 24 | 16,4 ± 5,2 | 8,3 |
| | 0,625/ 25 | 1 : 40 | 12 | 12 | 12 | 12 | 15 | 15 | 19 | 19 | 20 | 21 | 15,7 ± 3,7 | 7,6 |
| unbehandelte infizierte Kontrolltiere (IK) | — | — | 7 | 8 | 8 | 8 | 8 | 8 | 8 | 8 | 9 | 9 | 8,1 ± 0,6 | — |

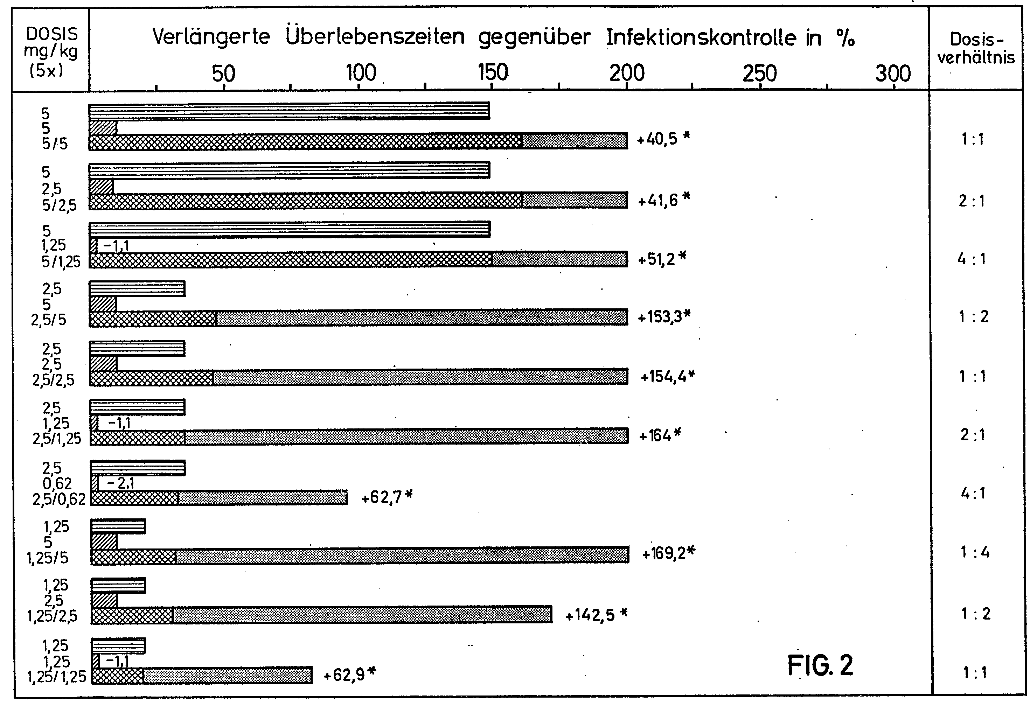

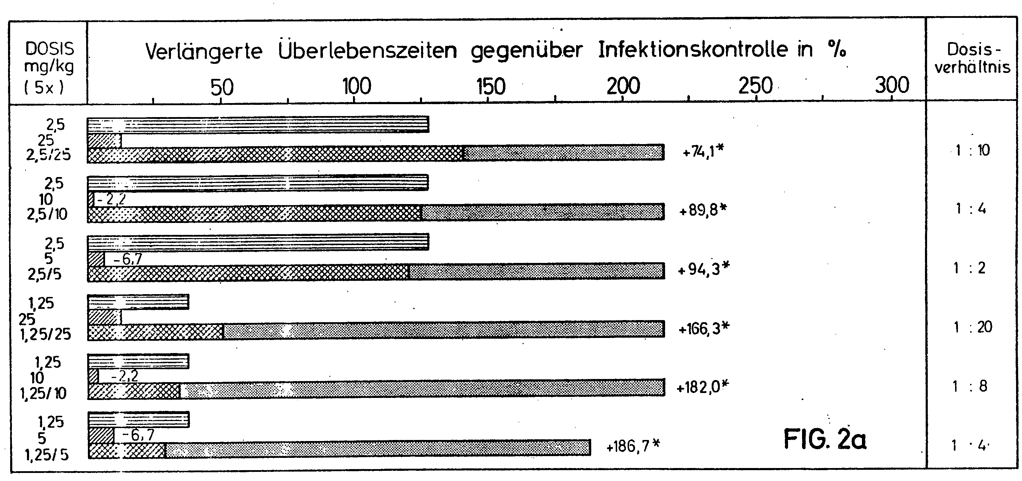

TABELLE 2: Synergistischer Effekt (überadditiver Anteil der Überlebenszeit in % — vgl. Fig. 2) nach Kombination von FLOXACRINE mit CHLOROQUINE auf Blutformon von Plasmodium berghei (arzneiempfindlicher Stamm) in der NMRI-Maus

| Präparat | Dosis mg/kg (5 ×) | Dosisverhältnis | Effekt gegen Blutformen von P. berghei, gemessen in überlebenszeiten (ÜZ) (Tage post infectionem) | | | | | | | | | | $\bar{x}$ , s | Verlängerte ÜZ $\bar{x}$ (Präp) −$\bar{x}$ (IK) |
|---|---|---|---|---|---|---|---|---|---|---|---|---|---|---|
| | | | beobachtete Werte | | | | | | | | | | | |
| Floxacrine | 5 | | 14 | 15 | 18 | 18 | 28 | 28 | 28 | 28 | 28 | 28 | 23,3 ± 6,2 | 13,9 |
| | 2,5 | | 11 | 12 | 12 | 12 | 12 | 12 | 13 | 13 | 13 | 17 | 12,7 ± 1,6 | 3,3 |
| | 1,25 | | 9 | 9 | 11 | 11 | 11 | 11 | 11 | 11 | 14 | 14 | 11,2 ± 1,7 | 1,8 |
| Chloroquine (Diphosphat) | 5 | | 9 | 9 | 9 | 9 | 10 | 10 | 10 | 10 | 13 | 14 | 10,3 ± 1,8 | 0,9 |
| | 2,5 | | 10 | 10 | 10 | 10 | 10 | 10 | 10 | 10 | 11 | 11 | 10,2 ± 0,4 | 0,8 |
| | 1,25 | | 9 | 9 | 9 | 9 | 9 | 9 | 9 | 10 | 10 | 10 | 9,3 ± 0,5 | −0,1 |
| | 0,62 | | 8 | 8 | 9 | 9 | 9 | 9 | 9 | 9 | 11 | 11 | 9,2 ± 1,0 | −0,2 |
| Floxacrine/ Chloroquine | 5 / 5 | 1 : 1 | 28 | 28 | 28 | 28 | 28 | 28 | 28 | 28 | 28 | 28 | 28 ± 0 | 18,6 |
| | 5 / 2,5 | 2 : 1 | 28 | 28 | 28 | 28 | 28 | 28 | 28 | 28 | 28 | 28 | 28 ± 0 | 18,6 |
| | 5 / 1,25 | 4 : 1 | 28 | 28 | 28 | 28 | 28 | 28 | 28 | 28 | 28 | 28 | 28 ± 0 | 18,6 |
| | 2,5/ 5 | 1 : 2 | 28 | 28 | 28 | 28 | 28 | 28 | 28 | 28 | 28 | 28 | 28 ± 0 | 18,6 |
| | 2,5/ 2,5 | 1 : 1 | 28 | 28 | 28 | 28 | 28 | 28 | 28 | 28 | 28 | 28 | 28 ± 0 | 18,6 |
| | 2,5/ 1,25 | 2 : 1 | 28 | 28 | 28 | 28 | 28 | 28 | 28 | 28 | 28 | 28 | 28 ± 0 | 18,6 |
| | 2,5/ 0,62 | 4 : 1 | 13 | 13 | 17 | 17 | 19 | 19 | 20 | 20 | 23 | 23 | 18,4 ± 3,5 | 9 |
| | 1,25/ 5 | 1 : 4 | 28 | 28 | 28 | 28 | 28 | 28 | 28 | 28 | 28 | 28 | 28 ± 0 | 18,6 |
| | 1,25/2,5 | 1 : 2 | 15 | 15 | 28 | 28 | 28 | 28 | 28 | 28 | 28 | 28 | 25,4 ± 5,5 | 16 |
| | 1,25/1,25 | 1 : 1 | 13 | 13 | 13 | 13 | 15 | 15 | 16 | 16 | 28 | 28 | 17 ± 5,9 | 7,6 |
| unbehandelte infizierte Kontrolltiere (IK) | — | — | 7 | 7 | 9 | 9 | 9 | 9 | 9 | 10 | 12 | 13 | 9,4 ± 1,9 | — |

TABELLE 2a: Synergistischer Effekt (überadditiver Anteil der Überlebenszeit in % — vgl. Fig. 2a) nach Kombination von FLOXACRINE mit CHLOROQUINE auf Blutformen von Plasmodium berghei (chloroquinresistenter Stamm) in der NMRI-Maus

| Präparat | Dosis mg/kg (5 ×) | Dosisver- hältnis | Effekt gegen Blutformen von P. berghei, gemessen in Überlebenszeiten (ÜZ) (Tage post infectionem) | | | | | | | | | | $\bar{x}$ , s | Verlängerte ÜZ $\bar{x}$ (Präp) −$\bar{x}$ (IK) |
|---|---|---|---|---|---|---|---|---|---|---|---|---|---|---|
| | | | beobachtete Werte | | | | | | | | | | | |
| Floxacrine | 2,5 | | 13 | 13 | 15 | 15 | 17 | 17 | 28 | 28 | 28 | 28 | 20,2 ± 6,8 | 11,3 |
| | 1,25 | | 8 | 8 | 13 | 13 | 13 | 13 | 13 | 13 | 13 | 13 | 12 ± 2,1 | 3,1 |
| Chloroquine (Diphosphat) | 25 | | 7 | 7 | 8 | 8 | 9 | 10 | 12 | 13 | 13 | 14 | 10,1 ± 2,7 | 1,2 |
| | 10 | | 6 | 6 | 7 | 8 | 8 | 9 | 10 | 10 | 10 | 13 | 8,7 ± 2,2 | −0,2 |
| | 5 | | 6 | 6 | 7 | 7 | 7 | 8 | 9 | 10 | 10 | 13 | 8,3 ± 2,2 | −0,6 |
| Floxacrine/ Chloroquine | 2,5/25 | 1 : 10 | 28 | 28 | 28 | 28 | 28 | 28 | 28 | 28 | 28 | 28 | 28 ± 0 | 19,1 |
| | 2,5/10 | 1 : 4 | 28 | 28 | 28 | 28 | 28 | 28 | 28 | 28 | 28 | 28 | 28 ± 0 | 19,1 |
| | 2,5/ 5 | 1 : 2 | 28 | 28 | 28 | 28 | 28 | 28 | 28 | 28 | 28 | 28 | 28 ± 0 | 19,1 |
| | 1,25/25 | 1 : 20 | 28 | 28 | 28 | 28 | 28 | 28 | 28 | 28 | 28 | 28 | 28 ± 0 | 19,1 |
| | 1,25/10 | 1 : 8 | 28 | 28 | 28 | 28 | 28 | 28 | 28 | 28 | 28 | 28 | 28 ± 0 | 19,1 |
| | 1,25/ 5 | 1 : 4 | 14 | 14 | 23 | 24 | 28 | 28 | 28 | 28 | 28 | 28 | 24,3 ± 5,7 | 15,4 |
| unbehandelte infizierte Kontrolltiere (IK) | — | — | 6 | 6 | 6 | 6 | 9 | 10 | 10 | 10 | 13 | 13 | 8,9 ± 2,8 | — |

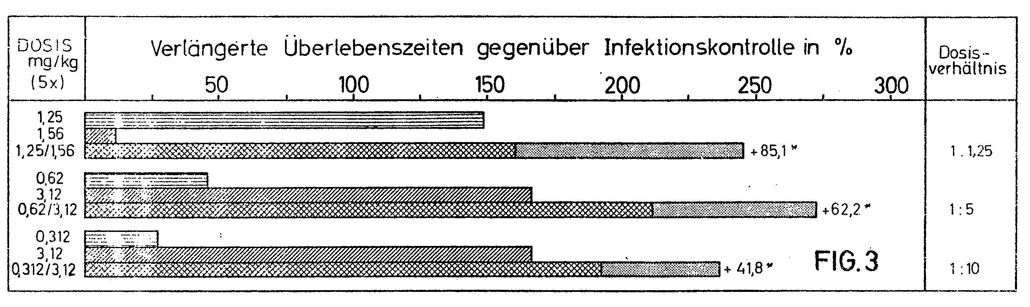

TABELLE 3: Synergistischer Effekt(überadditiver Anteil der Überlebenszeit in % — vgl. Fig. 3) nach Kombination von FLOXACRINE mit MEFLOQUINE auf Blutformen von Plasmodium berghei (arzneiempfindlicher Stamm) in der NMRI-Maus

| Präparat | Dosis mg/kg (5 ×) | Dosisver-hältnis | Effekt gegen Blutformen von P. berghei, gemessen in Überlebenszeiten (ÜZ) (Tage post infectionem) | | | | | | | | | | $\bar{x}$ , s | Verlängerte ÜZ $\bar{x}$ (Präp) −$\bar{x}$ (IK) |
|---|---|---|---|---|---|---|---|---|---|---|---|---|---|---|
| | | | beobachtete Werte | | | | | | | | | | | |
| Floxacrine | 1,25 | | 13 | 13 | 14 | 16 | 17 | 19 | 20 | 21 | 22 | 28 | 18,4 ± 4,6 | 11 |
| | 0,62 | | 8 | 9 | 10 | 10 | 10 | 11 | 11 | 11 | 13 | 14 | 10,7 ± 1,8 | 3,3 |
| | 0,312 | | 8 | 8 | 9 | 9 | 9 | 9 | 9 | 9 | 12 | 12 | 9,4 ± 1,4 | 2 |
| Mefloquine (Hydrochlorid) | 3,12 | | 13 | 13 | 17 | 17 | 17 | 21 | 21 | 22 | 28 | 28 | 19,7 ± 5,4 | 12,3 |
| | 1,56 | | 6 | 6 | 8 | 8 | 8 | 8 | 8 | 8 | 11 | 11 | 8,2 ± 1,7 | 0,8 |
| Floxacrine/ Mefloquine | 1,25 /1,56 | 1 : 1,25 | 15 | 16 | 28 | 28 | 28 | 28 | 28 | 28 | 28 | 28 | 25.5 ± 5,3 | 18,1 |
| | 0,62 /3,12 | 1 : 5 | 24 | 28 | 28 | 28 | 28 | 28 | 28 | 28 | 28 | 28 | 27,6 ± 1,3 | 20,2 |
| | 0,312/3,12 | 1 : 10 | 15 | 15 | 27 | 27 | 27 | 27 | 27 | 27 | 28 | 28 | 24,8 ± 5,2 | 17,4 |
| unbehandelte, infizierte Kontrolltiere (IK) | — | — | 6 | 7 | 7 | 7 | 7 | 7 | 8 | 8 | 8 | 9 | 7,4 ± 0,8 | — |

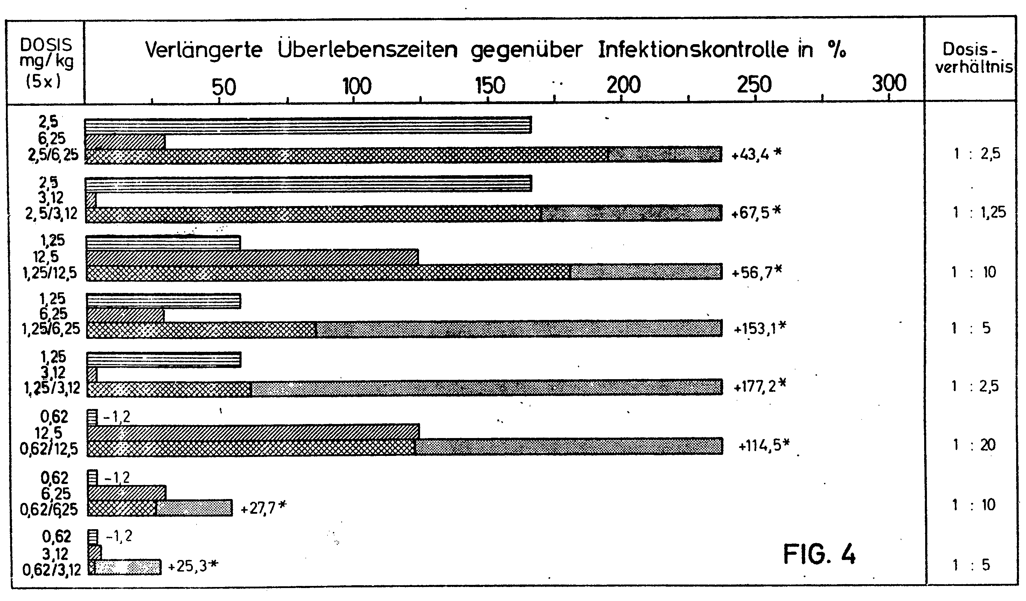

TABELLE 4: Synergistischer Effekt (überadditiver Anteil der Überlebenszeit in % — vgl. Fig. 4) nach Kombination von FLOXACRINE mit PRIMAQUINE auf Blutformen von Plasmodium berghei (arzneiempfindlicher Stamm) in der NMRI-Maus

| Präparat | Dosis mg/kg (5 ×) | Dosisver-hältnis | Effekt gegen Blutformen von P. berghei, gemessen in Überlebenszeiten (ÜZ) (Tage post infectionem) | | | | | | | | | beobachtete Werte | | $\bar{x}$ , s | Verlängerte ÜZ $\bar{x}$ (Präp) $-\bar{x}$ (IK) |
|---|---|---|---|---|---|---|---|---|---|---|---|---|---|---|---|
| Floxacrine | 2,5 | | 12 | 14 | 16 | 19 | 20 | 28 | 28 | 28 | 28 | 28 | 22,1 ± 6,6 | 13,8 |
| | 1,25 | | 8 | 8 | 12 | 12 | 13 | 13 | 14 | 16 | 17 | 17 | 13 ± 3,2 | 4,7 |
| | 0,62 | | 7 | 7 | 7 | 7 | 8 | 8 | 9 | 9 | 19 | 10 | 8,2 ± 1,2 | −0,1 |
| Primaquine (Diphosphat) | 12,5 | | 10 | 13 | 13 | 13 | 14 | 15 | 24 | 28 | 28 | 28 | 18,6 ± 7,4 | 10,3 |
| | 6,25 | | 10 | 10 | 10 | 10 | 10 | 10 | 11 | 11 | 12 | 12 | 10,6 ± 0,8 | 2,3 |
| | 3,12 | | 7 | 7 | 8 | 8 | 8 | 8 | 9 | 9 | 11 | 11 | 8,6 ± 1,4 | 0,3 |
| Floxacrine/ Primaquine | 2,5/6,25 | 1 : 2,5 | 28 | 28 | 28 | 28 | 28 | 28 | 28 | 28 | 28 | 28 | 28 ± 0 | 19,7 |
| | 2,5/3,12 | 1 : 1,25 | 28 | 28 | 28 | 28 | 28 | 28 | 28 | 28 | 28 | 28 | 28 ± 0 | 19,7 |
| | 1,25/12,5 | 1 : 10 | 28 | 28 | 28 | 28 | 28 | 28 | 28 | 28 | 28 | 28 | 28 ± 0 | 19,7 |
| | 1,25/ 6,25 | 1 : 5 | 28 | 28 | 28 | 28 | 28 | 28 | 28 | 28 | 28 | 28 | 28 ± 0 | 19,7 |
| | 1,25/ 3,12 | 1 : 2,5 | 28 | 28 | 28 | 28 | 28 | 28 | 28 | 28 | 28 | 28 | 28 ± 0 | 19,7 |
| | 0,62/12,5 | 1 : 20 | 28 | 28 | 28 | 28 | 28 | 28 | 28 | 28 | 28 | 28 | 28 ± 0 | 19,7 |
| | 0,62/ 6,25 | 1 : 10 | 10 | 10 | 12 | 12 | 12 | 12 | 13 | 13 | 17 | 17 | 12,8 ± 2,4 | 4,5 |
| | 0,62/ 3,12 | 1 : 5 | 8 | 8 | 9 | 9 | 11 | 11 | 12 | 12 | 13 | 13 | 10,6 ± 2 | 2,3 |
| unbehandelte infizierte Kontrolltiere (IK) | — | — | 7 | 8 | 8 | 8 | 8 | 8 | 9 | 9 | 9 | 9 | 8,3 ± 0,7 | — |

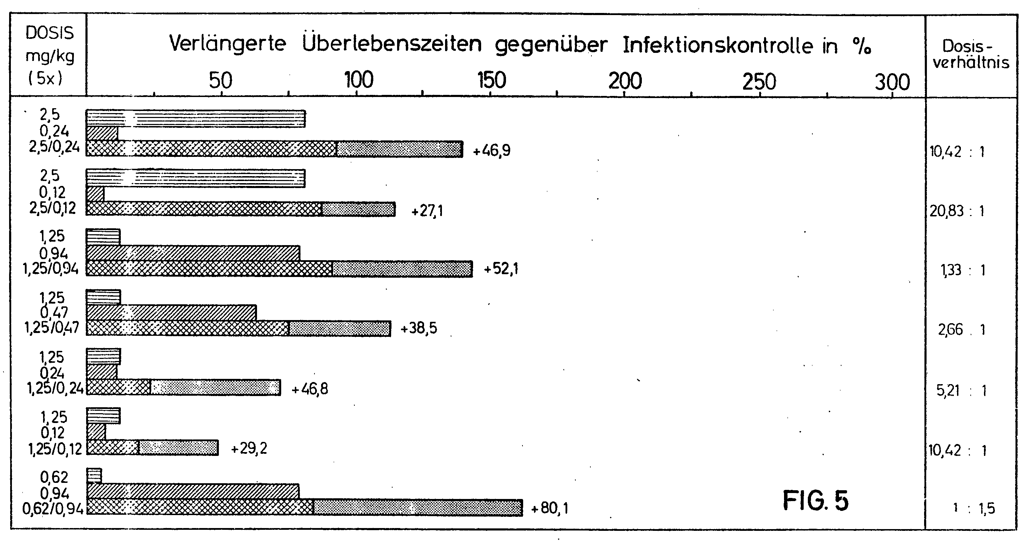

TABELLE 5: Synergistischer Effekt (uberadditiver Anteil der Überlebenszeit in % — vgl. Fig. 5) nach Kombination von FLOXACRINE mit PYRIMETHAMINE auf Blutformen von Plasmodium berghei (arzneiempfindlicher Stamm) in der NMRI-Maus

| Präparat | Dosis mg/kg (5 ×) | Dosisver- hältnis | Effekt gegen Blutformen von P. berghei, gemessen in Überlebenszeiten (ÜZ) (Tage post infectionem) | | | | | | | | | | $\bar{x}$ , s | Verlängerte ÜZ $\bar{x}$ (Präp) $-\bar{x}$ (IK) |
|---|---|---|---|---|---|---|---|---|---|---|---|---|---|---|
| | | | beobachtete Werte | | | | | | | | | | | |
| Floxacrine | 2,5 | | 13 | 13 | 13 | 13 | 14 | 14 | 19 | 19 | 28 | 28 | 17,4 ± 6,1 | 7,8 |
| | 1,25 | | 8 | 8 | 9 | 9 | 11 | 11 | 12 | 12 | 13 | 15 | 10,8 ± 2,3 | 1,2 |
| | 0,62 | | 8 | 8 | 8 | 9 | 9 | 10 | 11 | 11 | 13 | 13 | 10 ± 1,9 | 0,4 |
| Pyrimethamine | 0,94 | | 13 | 13 | 14 | 14 | 15 | 15 | 16 | 16 | 28 | 28 | 17,2 ± 5,8 | 7,6 |
| | 0,47 | | 10 | 10 | 13 | 13 | 13 | 14 | 15 | 16 | 23 | 28 | 15,5 ± 5,7 | 5,9 |
| | 0,24 | | 9 | 9 | 10 | 10 | 11 | 11 | 11 | 12 | 12 | 12 | 10,7 ± 1,2 | 1,1 |
| | 0,12 | | 6 | 9 | 9 | 9 | 10 | 11 | 11 | 11 | 13 | 13 | 10,2 ± 2,1 | 0,6 |
| | 2,5/0,24 | 10,42 : 1 | 16 | 16 | 19 | 19 | 24 | 24 | 28 | 28 | 28 | 28 | 23 ± 5,1 | 13,4 |
| | 2,5/0,12 | 20,83 : 1 | 15 | 15 | 16 | 16 | 16 | 16 | 28 | 28 | 28 | 28 | 20,6 ± 6,4 | 11 |
| Floxacrine/ Pyrimethamine | 1,25/0,94 | 1,33 : 1 | 15 | 16 | 17 | 18 | 28 | 28 | 28 | 28 | 28 | 28 | 23,4 ± 6 | 13,8 |
| | 1,25/0,47 | 2,66 : 1 | 14 | 14 | 15 | 15 | 17 | 17 | 28 | 28 | 28 | 28 | 20,4 ± 6,6 | 10,8 |
| | 1,25/0,24 | 5,21 : 1 | 10 | 12 | 15 | 15 | 15 | 15 | 17 | 17 | 24 | 24 | 16,4 ± 4,5 | 6,8 |
| | 1,25/0,12 | 10,42 : 1 | 12 | 12 | 13 | 13 | 15 | 15 | 15 | 15 | 16 | 16 | 14,2 ± 1,6 | 4,6 |
| | 0,62/0,94 | 1 : 1,5 | 14 | 15 | 28 | 28 | 28 | 28 | 28 | 28 | 28 | 28 | 25,3 ± 5,7 | 15,7 |
| unbehandelte, infizierte Kontrolltiere (IK) | — | — | 6 | 7 | 9 | 9 | 9 | 10 | 10 | 11 | 12 | 13 | 9,6 ± 2,1 | — |

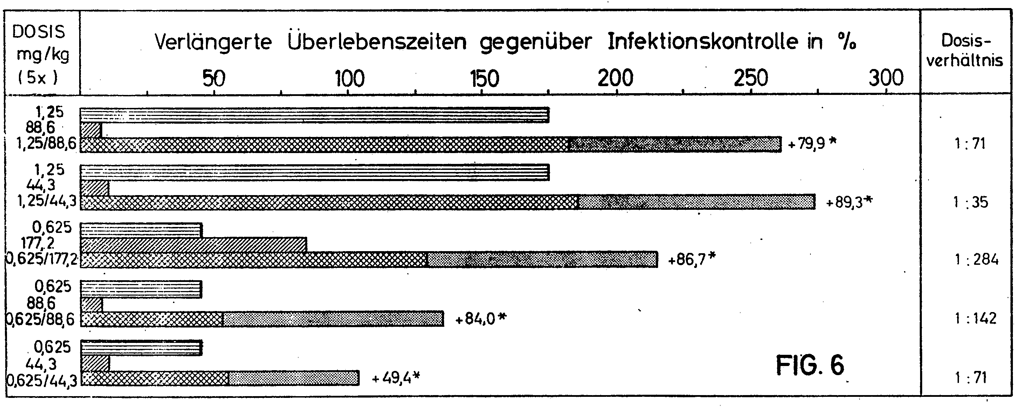

TABELLE 6: Synergistischer Effekt (überadditiver Anteil der Überlebenszeit in % — vgl. Fig. 6) nach Kombination von FLOXACRINE mit CYCLOGUANIL auf Blutformen von Plasmodium berghei (arzneiempfindlicher Stamm) in der NMRI-Maus

| Präparat | Dosis mg/kg (5 ×) | Dosisver-hältnis | Effekt gegen Blutformen von P. berghei, gemessen in Überlebenszeiten (ÜZ) (Tage post infectionem) | | | | | | | | | | $\bar{x}$ , s | Verlängerte ÜZ $\bar{x}$ (Präp) −$\bar{x}$ (IK) |
|---|---|---|---|---|---|---|---|---|---|---|---|---|---|---|
| | | | beobachtete Werte | | | | | | | | | | | |
| Floxacrine | 1,25 | | 14 | 14 | 19 | 19 | 20 | 20 | 22 | 22 | 28 | 28 | 20,6 ± 4,8 | 13,1 |
| | 0,625 | | 9 | 9 | 10 | 10 | 10 | 10 | 11 | 11 | 14 | 14 | 10,8 ± 1,8 | 3,3 |
| Cycloguanil pamoate | 177,2 | | 7 | 7 | 9 | 9 | 12 | 12 | 13 | 13 | 28 | 28 | 13,8 ± 7,8 | 6,3 |
| | 88,6 | | 7 | 7 | 8 | 8 | 8 | 8 | 8 | 8 | 9 | 9 | 8 ± 0,7 | 0,5 |
| | 44,3 | | 7 | 7 | 7 | 7 | 8 | 8 | 9 | 9 | 10 | 10 | 8,2 ± 1,2 | 0,7 |
| Floxacrine/ Cycloguanil | 1,25/ 88,6 | 1 : 71 | 23 | 24 | 28 | 28 | 28 | 28 | 28 | 28 | 28 | 28 | 27,1 ± 1,9 | 19,6 |
| | 1,25/ 44,3 | 1 : 35 | 28 | 28 | 28 | 28 | 28 | 28 | 28 | 28 | 28 | 28 | 28 ± 0 | 20,5 |
| | 0,625/177,2 | 1 : 284 | 17 | 17 | 22 | 22 | 23 | 23 | 28 | 28 | 28 | 28 | 23,6 ± 4,4 | 16,1 |
| | 0,625/ 88,6 | 1 : 142 | 13 | 13 | 14 | 14 | 19 | 19 | 19 | 19 | 23 | 23 | 17,6 ± 3,9 | 10,1 |
| | 0,625/ 44,3 | 1 : 71 | 13 | 13 | 13 | 13 | 16 | 16 | 17 | 17 | 17 | 17 | 15,2 ± 1,9 | 7,7 |
| unbehandelte, infizierte Kontrolltiere (IK) | — | — | 6 | 7 | 7 | 7 | 7 | 8 | 8 | 8 | 8 | 9 | 7,5 ± 0,9 | — |

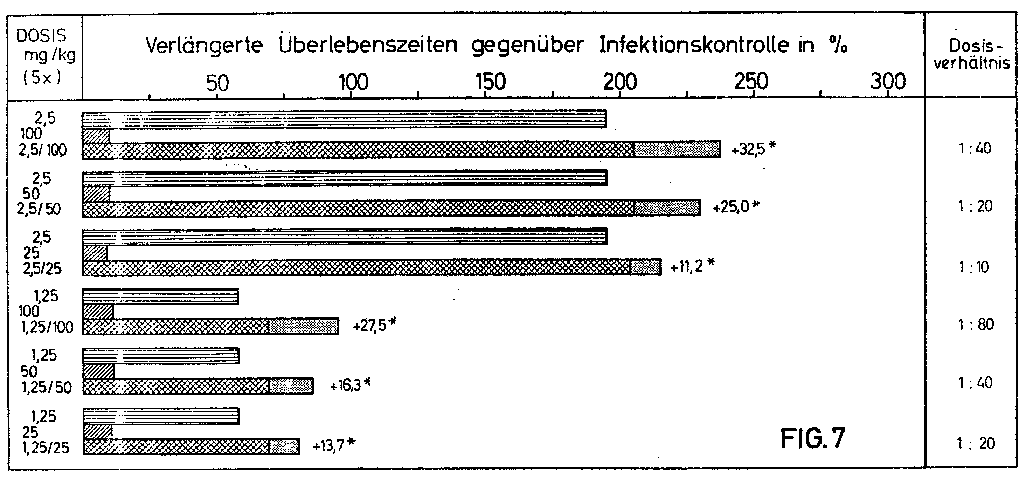

TABELLE 7: Synergistischer Effekt(überadditiver Anteil der Überlebenszeit in % — vgl. Fig. 7) nach Kombination von FLOXACRINE mit TRIMETHOPRIM auf Blutformen von Plasmodium berghei (arzneiempfindlicher Stamm) in der NMRI-Maus

| Präparat | Dosis mg/kg (5 ×) | Dosisver-hältnis | Effekt gegen Blutformen von P. berghei, gemessen in Überlebenszeiten (ÜZ) (Tage post infectionem) | | | | | | | | | | $\bar{x}$ , s | Verlängerte ÜZ $\bar{x}$ (Präp) $-\bar{x}$ (IX) |
|---|---|---|---|---|---|---|---|---|---|---|---|---|---|---|
| | | | beobachtete Werte | | | | | | | | | | | |
| Floxacrine | 2,5 | | 21 | 21 | 23 | 23 | 23 | 23 | 25 | 25 | 26 | 26 | 23,6 ± 1,8 | 15,6 |
| | 1,25 | | 9 | 9 | 12 | 12 | 12 | 12 | 15 | 15 | 16 | 16 | 12,8 ± 2,6 | 4,6 |
| Trimethoprim | 100 | | 8 | 8 | 8 | 8 | 9 | 9 | 9 | 9 | 10 | 10 | 8,8 ± 0,8 | 0,8 |
| | 50 | | 7 | 7 | 7 | 7 | 9 | 9 | 10 | 10 | 11 | 11 | 8,8 ± 1,7 | 0,8 |
| | 25 | | 7 | 7 | 7 | 7 | 9 | 9 | 10 | 10 | 10 | 11 | 8,7 ± 1,6 | 0,7 |
| Floxacrine Trimethoprim | 2,5/100 | 1 : 40 | 23 | 23 | 28 | 28 | 28 | 28 | 28 | 28 | 28 | 28 | 27 ± 2,1 | 19 |
| | 2,5/ 50 | 1 : 20 | 20 | 20 | 28 | 28 | 28 | 28 | 28 | 28 | 28 | 28 | 26,4 ± 3,4 | 18,4 |
| | 2,5/ 25 | 1 : 10 | 20 | 20 | 22 | 22 | 28 | 28 | 28 | 28 | 28 | 28 | 25,2 ± 3,7 | 17,2 |
| | 1,25/100 | 1 : 80 | 13 | 13 | 15 | 15 | 16 | 16 | 17 | 17 | 17 | 17 | 15,6 ± 1,6 | 7,6 |
| | 1,25/ 50 | 1 : 40 | 13 | 13 | 13 | 13 | 15 | 15 | 15 | 16 | 17 | 17 | 14,7 ± 1,6 | 6,7 |
| | 1,25/ 25 | 1 : 20 | 11 | 11 | 13 | 13 | 14 | 14 | 17 | 17 | 17 | 17 | 14,4 ± 2,5 | 6,4 |
| unbehandelte infizierte Kontrolltiere (IK) | — | — | 6 | 6 | 6 | 7 | 8 | 9 | 9 | 9 | 10 | 10 | 8 ± 1,6 | — |

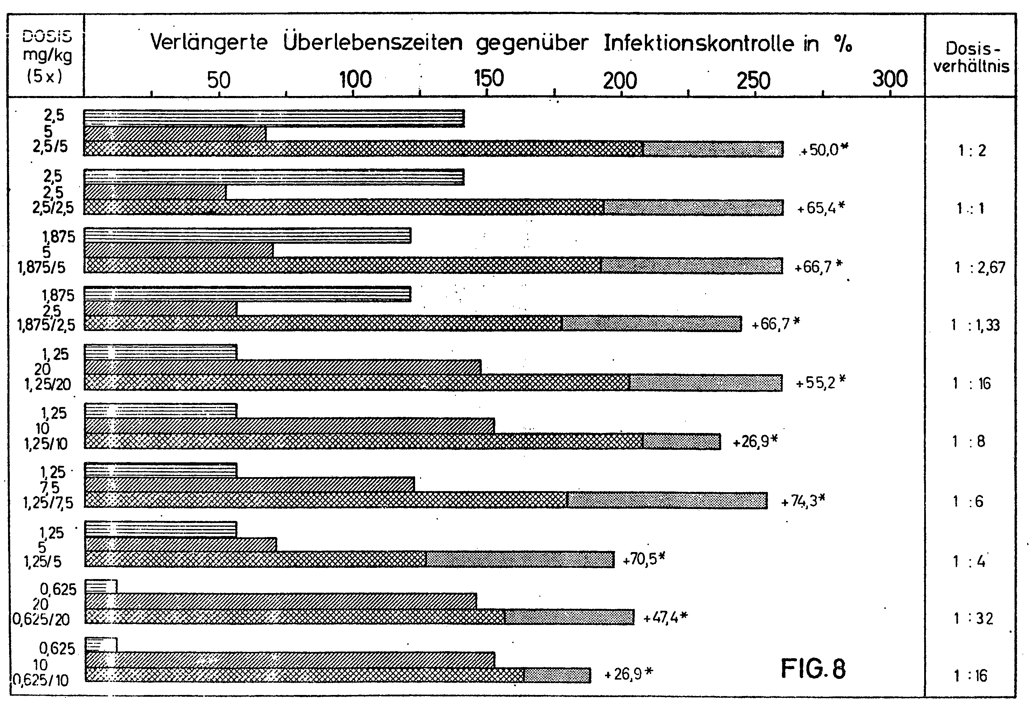

TABELLE 8: Synergistischer Effekt (überadditiver Anteil der Überlebenszeit in % — vgl. Fig. 8) nach Kombination von FLOXACRINE mit SULFADOXINE auf Blutformen von Plasmodium berghei (arzneiempfindlicher Stamm) in der NMRI-Maus

| Präparat | Dosis mg/kg (5 ×) | Dosisver- hältnis | Effekt gegen Blutformen von P. berghei, gemessen in Überlebenszeiten (ÜZ) (Tage post infectionem) beobachtete Werte | | | | | | | | | | $\bar{x}$ , s | Verlängerte ÜZ $\bar{x}$ (Präp) −$\bar{x}$ (IK) |
|---|---|---|---|---|---|---|---|---|---|---|---|---|---|---|
| Floxacrine | 2,5 | | 14 | 14 | 14 | 15 | 16 | 16 | 18 | 23 | 28 | 28 | 18,6 ± 5,6 | 10,8 |
| | 1,875 | | 13 | 13 | 14 | 14 | 15 | 15 | 16 | 19 | 26 | 28 | 17,3 ± 5,4 | 9,5 |
| | 1,25 | | 8 | 9 | 11 | 12 | 12 | 12 | 14 | 14 | 15 | 15 | 12,2 ± 2,4 | 4,4 |
| | 0,625 | | 6 | 7 | 7 | 8 | 9 | 9 | 9 | 9 | 10 | 11 | 8,5 ± 1,5 | 0,7 |
| Sulfadoxine | 20 | | 13 | 14 | 14 | 15 | 17 | 17 | 19 | 28 | 28 | 28 | 19,3 ± 6,3 | 11,5 |
| | 10 | | 13 | 14 | 15 | 16 | 17 | 20 | 23 | 23 | 28 | 28 | 19,7 ± 5,6 | 11,9 |
| | 7,5 | | 12 | 12 | 12 | 13 | 14 | 15 | 16 | 24 | 28 | 28 | 17,4 ± 6,6 | 9,6 |
| | 5 | | 11 | 12 | 12 | 12 | 13 | 13 | 14 | 14 | 15 | 17 | 13,3 ± 1,8 | 5,5 |
| | 2,5 | | 10 | 10 | 10 | 10 | 11 | 11 | 12 | 15 | 15 | 17 | 12,1 ± 2,6 | 4,3 |
| Floxacrine/ Sulfadoxine | 2,5/ 5 | 1 : 2 | 28 | 28 | 28 | 28 | 28 | 28 | 28 | 28 | 28 | 28 | 28 ± 0 | 20,2 |
| | 2,5/ 2,5 | 1 : 1 | 28 | 28 | 28 | 28 | 28 | 28 | 28 | 28 | 28 | 28 | 28 ± 0 | 20,2 |
| | 1,875/ 5 | 1 : 2,67 | 28 | 28 | 28 | 28 | 28 | 28 | 28 | 28 | 28 | 28 | 28 ± 0 | 20,2 |
| | 1,875/ 2,5 | 1 : 1,33 | 20 | 24 | 28 | 28 | 28 | 28 | 28 | 28 | 28 | 28 | 26,8 ± 2,7 | 19 |
| | 1,25/20 | 1 : 16 | 28 | 28 | 28 | 28 | 28 | 28 | 28 | 28 | 28 | 28 | 28 ± 0 | 20,2 |
| | 1,25/10 | 1 : 8 | 17 | 21 | 28 | 28 | 28 | 28 | 28 | 28 | 28 | 28 | 26,2 ± 3,9 | 18,4 |
| | 1,25/ 7,5 | 1 : 6 | 26 | 26 | 28 | 28 | 28 | 28 | 28 | 28 | 28 | 28 | 27,6 ± 0,8 | 19,8 |
| | 1,25/ 5 | 1 : 4 | 15 | 15 | 17 | 17 | 28 | 28 | 28 | 28 | 28 | 28 | 23,2 ± 6,2 | 15,4 |
| | 0,625/20 | 1 : 32 | 14 | 16 | 21 | 24 | 24 | 26 | 28 | 28 | 28 | 28 | 23,7 ± 5,2 | 15,9 |
| | 0,625/10 | 1 : 16 | 14 | 15 | 20 | 22 | 23 | 24 | 26 | 26 | 27 | 28 | 22,5 ± 4,9 | 14,7 |
| unbehandelte, infizierte Kontrolltiere (IK) | — | — | 7 | 7 | 7 | 7 | 7 | 8 | 8 | 8 | 9 | 10 | 7,8 ± 1 | — |

0001796

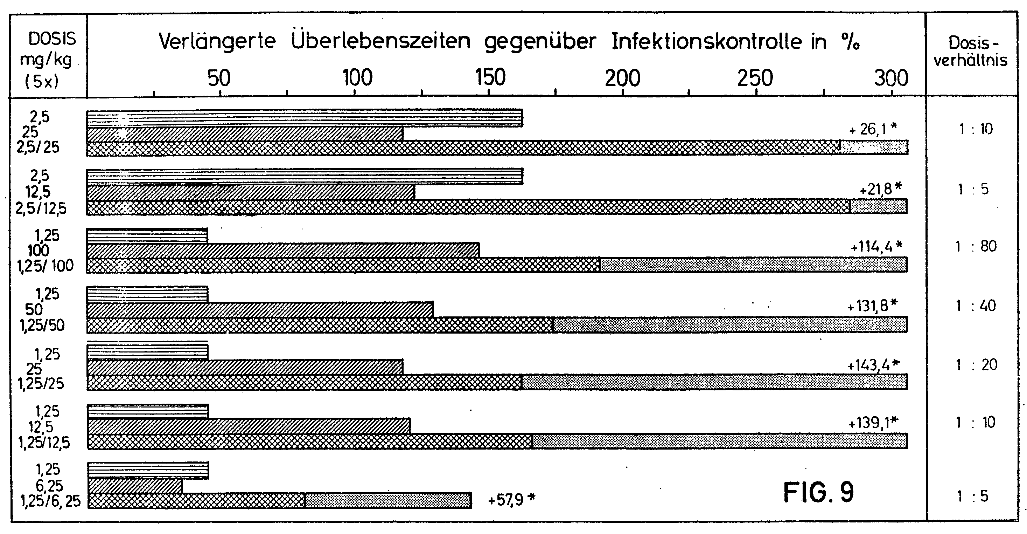

TABELLE 9: Synergistischer Effekt (überadditiver Anteil der Überlebenszeit in % — vgl. Fig. 9) nach Kombination von FLOXACRINE mit DAPSONE auf Blutformen von Plasmodium berghei (arzneiempfindlicher Stamm) in der NMRI-Maus

| Präparat | Dosis mg/kg (5 ×) | Dosisverhältnis | Effekt gegen Blutformen von P. berghei, gemessen in Überlebenszeiten (ÜZ) (Tage post infectionem) beobachtete Werte | | | | | | | | | | $\bar{x}$ , s | Verlängerte ÜZ $\bar{x}$ (Präp) $-\bar{x}$ (IK) |
|---|---|---|---|---|---|---|---|---|---|---|---|---|---|---|
| Floxacrine | 2,5 | | 14 | 14 | 15 | 15 | 16 | 17 | 17 | 17 | 28 | 28 | 18,1 ± 5,3 | 11,2 |
|  | 1,25 | | 8 | 8 | 9 | 9 | 9 | 10 | 11 | 11 | 11 | 14 | 10 ± 1,8 | 3,1 |
| Dapsone | 100 | | 12 | 13 | 13 | 14 | 15 | 15 | 16 | 16 | 28 | 28 | 17 ± 5,9 | 10,1 |
|  | 50 | | 12 | 12 | 13 | 13 | 13 | 15 | 16 | 18 | 22 | 24 | 15,8 ± 4,3 | 8,9 |
|  | 25 | | 12 | 12 | 13 | 13 | 13 | 15 | 16 | 17 | 19 | 20 | 15 ± 2,9 | 8,1 |
|  | 12,5 | | 12 | 13 | 13 | 13 | 15 | 15 | 15 | 16 | 17 | 24 | 15,3 ± 3,4 | 8,4 |
|  | 6,25 | | 8 | 8 | 8 | 8 | 9 | 10 | 10 | 10 | 11 | 11 | 9,3 ± 1,3 | 2,4 |
| Floxacrine/ Dapsone | 2,5/25 | 1 : 10 | 28 | 28 | 28 | 28 | 28 | 28 | 28 | 28 | 28 | 28 | 28 ± 0 | 21,1 |
|  | 2,5/12,5 | 1 : 5 | 28 | 28 | 28 | 28 | 28 | 28 | 28 | 28 | 28 | 28 | 28 ± 0 | 21,1 |
|  | 1,25/100 | 1 : 80 | 28 | 28 | 28 | 28 | 28 | 28 | 28 | 28 | 28 | 28 | 28 ± 0 | 21,1 |
|  | 1,25/ 50 | 1 : 40 | 28 | 28 | 28 | 28 | 28 | 28 | 28 | 28 | 28 | 28 | 28 ± 0 | 21,1 |
|  | 1,25/ 25 | 1 : 20 | 28 | 28 | 28 | 28 | 28 | 28 | 28 | 28 | 28 | 28 | 28 ± 0 | 21,1 |
|  | 1,25/ 12,5 | 1 : 10 | 28 | 28 | 28 | 28 | 28 | 28 | 28 | 28 | 28 | 28 | 28 ± 0 | 21,1 |
|  | 1,25/ 6,25 | 1 : 5 | 12 | 12 | 13 | 13 | 14 | 14 | 15 | 15 | 28 | 28 | 16,4 ± 6,2 | 9,5 |
| unbehandelte infizierte Kontrolltiere (IK) | — | — | 6 | 6 | 6 | 6 | 7 | 7 | 7 | 7 | 8 | 9 | 6,9 ± 1 | — |

**0 001 796**

Patentansprüche

1. Mittel gegen Malaria, gekennzeichnet durch einen Gehalt an 7-Chlor-10-hydroxy-3-(4-trifluormethylphenyl)-3,4-dihydroacridin-1,9-(2H,10H)-dion (Floxacrin) oder einem seiner Salze mit einer physiologisch verträglichen Säure oder Base, in Mischung mit
   a) 6-Methoxy-$\alpha$-(5-vinyl-2-chinuclidinyl)-4-chinolinmethanol (Chinin),
   b) 7-Chlor-4-(diäthylamino-1-methyl-butylamino)-chinolin (Chloroquine),
   c) $\alpha$-(2-Piperidyl)-2,8-bis(trifluormethyl)-4-chinolin-methanol (Mefloquine),
   d) 8-(4-Amino-1-methylbutylamino)-6-methoxy-chinolin (Primaquine),
   e) 2,4-Diamino-5-p-chlorphenyl-6-äthylpyrimidin (Pyrimethamine),
   f) 4,6-Diamino-1-(p-chlorphenyl)-1,2-dihydro-2,2-dimethyl-s-triazin (Cycloguanil),
   g) 2,4-Diamino-5-(3,4,5-trimethoxybenzyl)-pyrimidin (Trimethoprim),
   h) N'-(5,6-Dimethoxy-4-pyrimidyl)-sulfanilamid (Sulfadoxine) oder
   i) 4,4'-Diaminodiphenylsulfon (Dapsone),
oder mit einem Salz der Verbindungen a) bis i) mit einer physiologisch verträglichen Säure oder Base, in einem Gewichtsverhältnis zwischen 25:1 und 1:300 (Floxacrin zu a) bis i)), mit einem pharmazeutisch üblichen Träger und/oder Konstituens.

2. Mittel nach Anspruch 1, gekennzeichnet durch eine Mischung von Floxacrine mit Chinin in einem Gewichtsverhältnis zwischen 1:10 und 1:160.

3. Mittel nach Anspruch 1, gekennzeichnet durch eine Mischung von Floxacrine mit Chloroquine in einem Gewichtsverhältnis zwischen 4:1 und 1:20.

4. Mittel nach Anspruch 1, gekennzeichnet durch eine Mischung von Floxacrine mit Mefloquine in einem Gewichtsverhältnis zwischen 1:1,25 und 1:10.

5. Mittel nach Anspruch 1, gekennzeichnet durch eine Mischung von Floxacrine mit Primaquine in einem Gewichtsverhältnis zwischen 1:1,25 und 1:20.

6. Mittel nach Anspruch 1, gekennzeichnet durch eine Mischung von Floxacrine mit Pyrimethamine in einem Gewichtsverhältnis zwischen 21:1 und 1:1,5.

7. Mittel nach Anspruch 1, gekennzeichnet durch eine Mischung von Floxacrine mit Cycloguanil in einem Gewichtsverhältnis zwischen 1:35 und 1:284.

8. Mittel nach Anspruch 1, gekennzeichnet durch eine Mischung von Floxacrine mit Trimethoprim in einem Gewichtsverhältnis zwischen 1:10 und 1:80.

9. Mittel nach Anspruch 1, gekennzeichnet durch eine Mischung von Floxacrine mit Sulfadoxine in einem Gewichtsverhältnis zwischen 1:1 und 1:32.

10. Mittel nach Anspruch 1, gekennzeichnet durch eine Mischung von Floxacrine mit Dapsone in einem Gewichtsverhältnis zwischen 1:5 und 1:80.

11. Mittel nach Anspruch 3, 4, 5, 6 oder 9 in einem Gewichtsverhältnis zwischen 21:1 und 1:32.

12. Mittel nach Anspruch 2, 7, 8 oder 10, dadurch gekennzeichnet, daß es in einer Gesamtdosis pro Verabreichung von 7,5—178 mg/kg Körpergewicht vorliegt.

13. Mittel nach Anspruch 2, 7, 8 oder 10, dadurch gekennzeichnet, daß es in einer Gesamtdosis pro Verabreichung von 26,25—102,5 mg/kg Körpergewicht vorliegt.

14. Mittel nach Anspruch 3, 4, 5, 6 oder 9, dadurch gekennzeichnet, daß es in einer Gesamtdosis pro Verabreichung von 1,37—21,25 mg/kg Körpergewicht vorliegt.

15. Mittel nach Anspruch 3, 4, 5, 6 oder 9, dadurch gekennzeichnet, daß es in einer Gesamtdosis pro Verabreichung von 1,56—11,25 mg/kg Körpergewicht vorliegt.

Revendications

1. Médicament antipaludéen, caractérisé en ce qu'il contient de la 7-chloro-10-hydroxy-3-(4-trifluorométhylphényl)-3,4-dihydroacridine-1,9-(2H,10H)-dione (floxacrine) ou un de ses sels avec un acide ou une base physiologiquement acceptable, en mélange avec:
   a) le 6-méthoxy-$\alpha$-(5-vinyl-2-quinuclidinyl)-4-quinoléine-méthanol (quinine),
   b) la 7-chloro-4-(diéthylamino-1-méthyl-butylamino)-quinoléine (chloroquine),
   c) 1'$\alpha$-(2-pipéridyl)-2,8-bis(trifluorométhyl)-4-quinoléine méthanol (méfloquine),
   d) la 8-(4-amino-1-méthylbutylamino)-6-méthoxy-quinoléine (primaquine),
   e) la 2,4-diamino-5-p-chlorophényl-6-éthylpyrimidine (pyriméthamine),
   f) la 4,6-diamino-1-(p-chlorophényl)-1,2-dihydro-2,2-diméthyl-s-triazine (cycloguanil),
   g) la 2,4-diamino-5-(3,4,5-triméthoxybenzyl)-pyrimidine (triméthoprime),
   h) le N'-(5,6-diméthoxy-4-pyrimidyl)-sulfanilamide (sulfadoxine) ou,
   i) la 4,4'-diaminodiphénylsulfone (dapsone),
ou avec un sel des composès a) à i) avec un acide ou une base physiologiquement acceptable, dans un rapport pondéral compris entre 25:1 et 1:300 (floxacrine à a) à i) avec un véhicule et/ou des constituants pharmaceutiquement usuels.

2. Médicament selon la revendication 1, caractérisé en ce qu'il contient un mélange de floxacrine et de quinine dans un rapport pondéral compris entre 1:10 et 1:160.

3. Médicament selon la revendication 1, caractérisé en ce qu'il contient un mélange de floxacrine

15

et de chloroquine dans un rapport pondéral compris entre 4:1 et 1:20.

4. Médicament selon la revendication 1, caractérisé en ce qu'il contient un mélange de floxacrine et de méfloquine dans un rapport pondéral compris entre 1:1,25 et 1:10.

5. Médicament selon la revendication 1, caractérisé en ce qu'il contient un mélange de floxacrine et de primaquine dans un rapport pondéral compris entre 1:1,25 et 1:20.

6. Médicament selon la revendication 1, caractérisé en ce qu'il contient un mélange de floxacrine et de pyriméthamine dans un rapport pondéral compris entre 21:1 et 1:1.5.

7. Médicament selon la revendication 1, caractérisé en ce qu'il contient un mélange de floxacrine et de cycloguanil dans un rapport pondéral compris entre 1:35 et 1:284.

8. Médicament selon la revendication 1, caractérisé en ce qu'il contient un mélange de floxacrine et de triméthoprime dans un rapport pondéral compris entre 1:10 et 1:80.

9. Médicament selon la revendication 1, caractérisé en ce qu'il contient un mélange de floxacrine et de sulfadoxine dans un rapport pondéral compris entre 1:1 et 1:32.

10. Médicament selon la revendication 1, caractérisé en ce qu'il contient un mélange de floxacrine et de dapsone dans un rapport pondéral compris entre 1:5 et 1:80.

11. Médicament selon l'une quelconque des revendications 3, 4, 5, 6 ou 9, caractérisé en ce que le rapport pondéral est compris entre 21:1 et 1:32.

12. Médicament selon l'une quelconque des revendications 2, 7, 8 ou 10, caractérisé en ce qu'il est présent en une dose globale, par administration, de 7,5 à 178 mg/kg de poids corporel.

13. Médicament selon l'une quelconque des revendications 2, 7, 8 ou 10, caractérisé en ce qu'il est présent en une dose globale, par administration, de 26,25 à 102,5 mg/kg de poids corporel.

14. Médicament selon l'une quelconque des revendications 3, 4, 5, 6 ou 9, caractérisé en ce qu'il est présent en une dose globale par administration de 1,37 à 21,25 mg/kg de poids corporel.

15. Médicament selon l'une quelconque des revendications 3, 4, 5, 6 ou 9, caractérisé en ce qu'il est présent en une dose globale, par administration, de 1,56 à 11,25 mg/kg de poids corporel.

## Claims

1. Antimalarial composition containing as the active ingredient a mixture of 7-chloro-10-hydroxy-3-(4-trifluoromethyl-phenyl)-3,4-dihydroacridino-1,9-(2H,10H)-dione (Floxacrine) or a salt of this compound with a physiologically compatible acid or base, with

a) 6-methoxy-$\alpha$-(5-vinyl-2-quinuclidinyl)-4-quinoline-methanol (Quinine),
b) 7-chloro-4-(diethylamino-1-methyl-butylamino)-quinoline (Chloroquine),
c) $\alpha$-(2-piperidyl)-2,8-bis(trifluoromethyl)-4-quinoline-methanol (Mefloquine),
d) 8-(4-amino-1-methylbutylamino)-6-methoxy-quinoline (Primaquine),
e) 2,4-diamino-5-p-chlorophenyl-6-ethylpyrimidine (Pyrimethamine),
f) 4,6-dimaino-1-(p-chlorophenyl)-1,2-dihydro-2,2-dimethyl-s-triazine (Cycloguanil),
g) 2,4-diamino-5-(3,4,5-trimethoxybenzyl)-pyrimidine (Trimethoprim),
h) N'-(5,6-dimethoxy-4-pyrimidyl)-sulfanilamide (Sulfadoxine), or
i) 4,4'-diaminodiphenylsulfone (Dapsone),

or with a salt of a compound sub a) to i) with a physiologically compatible acid or base, the mixtures being in a proportion by weight or between 25:1 and 1:300 Floxacrine to compound sub a) to i), in admixture with a pharmaceutically acceptable carrier and/or adjuvant.

2. Composition as defined in claim 1, containing as the active ingredient a mixture of Floxacrine and Quinine in a proportion by weight of between 1:10 and 1:160.

3. Composition as defined in claim 1, containing as the active ingredient a mixture of Floxacrine and Chloroquine in a proportion by weight of between 4:1 and 1:20.

4. Composition as defined in claim 1, containing as the active ingredient a mixture of Floxacrine and Mefloquine in a proportion by weight of between 1:1.25 and 1:10.

5. Composition as defined in claim 1, containing as the active ingredient a mixture of Floxacrine and Primaquine in a proportion by weight of between 1:1.25 and 1:20.

6. Composition as defined in claim 1, containing as the active ingredient a mixture of Floxacrine and Pyrimethamine in a proportion by weight of between 21:1 and 1:1.5.

7. Composition as defined in claim 1, containing as the active ingredient a mixture of Floxacrine and Cycloguanil in a proportion by weight of between 1:35 and 1:284.

8. Composition as defined in claim 1, containing as the active ingredient a mixture of Floxacrine and Trimethoprim in a proportion by weight of between 1:10 and 1:80.

9. Composition as defined in claim 1, containing as the active ingredient a mixture of Floxacrine and Sulfadoxine in a proportion by weight of between 1:1 and 1:32.

10. Composition as defined in claim 1 or 2, containing as the active ingredient a mixture of Floxacrine and Dapsone in a proportion by weight of between 1:5 and 1:80.

11. Composition as defined in claims 3, 4, 5, 6 or 9 in a proportion by weight of between 21:1 and 1:32.

12. Composition as defined in claims 2, 7, 8 or 10, which is administered in a total dose of 7.5 to 178 mg/kg of body weight.

16

0001796

13. Composition as defined in claims 2, 7, 8 or 10, which is administered in a total dose of 26.25 to 102.5 mg/kg of body weight.

14. Composition as defined in claims 3, 4, 5, 6 or 9, which is administered in a total dose of 1.37 to 27.5 mg/kg of body weight.

15. Composition as defined in claims 3, 4, 5, 6 or 9, which is administered in a total dose of 1.56 to 11.25 mg/kg of body weight.

FIG. 1

0001796

Verlängerte Überlebenszeiten gegenüber Infektionskontrolle in %

| DOSIS mg/kg (5x) | Wert | Dosis-verhältnis |
|---|---|---|
| 5 / 5 / 5/5 | +40,5 * | 1 : 1 |
| 5 / 2,5 / 5/2,5 | +41,6 * | 2 : 1 |
| 5 / 1,25 / 5/1,25 | −1,1 ; +51,2 * | 4 : 1 |
| 2,5 / 5 / 2,5/5 | +153,3 * | 1 : 2 |
| 2,5 / 2,5 / 2,5/2,5 | +154,4 * | 1 : 1 |
| 2,5 / 1,25 / 2,5/1,25 | −1,1 ; +164 * | 2 : 1 |
| 2,5 / 0,62 / 2,5/0,62 | −2,1 ; +62,7 * | 4 : 1 |
| 1,25 / 5 / 1,25/5 | +169,2 * | 1 : 4 |
| 1,25 / 2,5 / 1,25/2,5 | +142,5 * | 1 : 2 |
| 1,25 / 1,25 / 1,25/1,25 | −1,1 ; +62,9 * | 1 : 1 |

Skala: 50 100 150 200 250 300

FIG. 2

| DOSIS mg/kg (5x) | Verlängerte Überlebenszeiten gegenüber Infektionskontrolle in % | Dosis-verhältnis |
|---|---|---|
| 2,5 / 25 / 2,5/25 | +74,1* | 1 : 10 |
| 2,5 / 10 / 2,5/10 | -2,2 / +89,8* | 1 : 4 |
| 2,5 / 5 / 2,5/5 | -6,7 / +94,3* | 1 : 2 |
| 1,25 / 25 / 1,25/25 | +166,3* | 1 : 20 |
| 1,25 / 10 / 1,25/10 | -2,2 / +182,0* | 1 : 8 |
| 1,25 / 5 / 1,25/5 | -6,7 / +186,7* | 1 : 4 |

x-axis: 50   100   150   200   250   300

FIG. 2a

| DOSIS mg/kg (5x) | Verlängerte Überlebenszeiten gegenüber Infektionskontrolle in % | Dosis-verhältnis |
|---|---|---|
| 1,25 1,56 1,25/1,56 | +85,1 * | 1 . 1,25 |
| 0,62 3,12 0,62/3,12 | +62,2 * | 1 : 5 |
| 0,312 3,12 0,312/3,12 | +41,8 * | 1 : 10 |

FIG. 3

0 001 796

| DOSIS mg/kg (5x) | Verlängerte Überlebenszeiten gegenüber Infektionskontrolle in % | Dosis-verhältnis |
|---|---|---|
| 2,5 / 6,25 / 2,5/6,25 | +43,4 * | 1 : 2,5 |
| 2,5 / 3,12 / 2,5/3,12 | +67,5 * | 1 : 1,25 |
| 1,25 / 12,5 / 1,25/12,5 | +56,7 * | 1 : 10 |
| 1,25 / 6,25 / 1,25/6,25 | +153,1 * | 1 : 5 |
| 1,25 / 3,12 / 1,25/3,12 | +177,2 * | 1 : 2,5 |
| 0,62 / 12,5 / 0,62/12,5 | −1,2 / +114,5 * | 1 : 20 |
| 0,62 / 6,25 / 0,62/6,25 | −1,2 / +27,7 * | 1 : 10 |
| 0,62 / 3,12 / 0,62/3,12 | −1,2 / +25,3 * | 1 : 5 |

Skala: 50 100 150 200 250 300

FIG. 4

| DOSIS mg/kg (5x) | Verlängerte Überlebenszeiten gegenüber Infektionskontrolle in % | Dosis-verhältnis |
|---|---|---|

Verlängerte Überlebenszeiten gegenüber Infektionskontrolle in %

50    100    150    200    250    300

2,5
0,24
2,5/0,24    +46,9    10,42 : 1

2,5
0,12
2,5/0,12    +27,1    20,83 : 1

1,25
0,94
1,25/0,94    +52,1    1,33 : 1

1,25
0,47
1,25/0,47    +38,5    2,66 . 1

1,25
0,24
1,25/0,24    +46,8    5,21 : 1

1,25
0,12
1,25/0,12    +29,2    10,42 : 1

0,62
0,94
0,62/0,94    +80,1    1 : 1,5

**FIG. 5**

0 001 796

FIG. 6

DOSIS mg/kg (5x) — Verlängerte Überlebenszeiten gegenüber Infektionskontrolle in % — Dosis-verhältnis

| DOSIS mg/kg (5x) | Verlängerte Überlebenszeiten | Dosis-verhältnis |
|---|---|---|
| 1,25 / 88,6 / 1,25/88,6 | +79,9 * | 1 : 71 |
| 1,25 / 44,3 / 1,25/44,3 | +89,3* | 1 : 35 |
| 0,625 / 177,2 / 0,625/177,2 | +86,7* | 1 : 284 |
| 0,625 / 88,6 / 0,625/88,6 | +84,0* | 1 : 142 |
| 0,625 / 44,3 / 0,625/44,3 | +49,4* | 1 : 71 |

0001796

| DOSIS mg/kg (5x) | Verlängerte Überlebenszeiten gegenüber Infektionskontrolle in % | Dosis-verhältnis |
|---|---|---|
| 2,5 / 100 / 2,5/100 | +32,5 * | 1:40 |
| 2,5 / 50 / 2,5/50 | +25,0 * | 1:20 |
| 2,5 / 25 / 2,5/25 | +11,2 * | 1:10 |
| 1,25 / 100 / 1,25/100 | +27,5 * | 1:80 |
| 1,25 / 50 / 1,25/50 | +16,3 * | 1:40 |
| 1,25 / 25 / 1,25/25 | +13,7 * | 1:20 |

FIG. 7

0001796

| DOSIS mg/kg (5x) | Verlängerte Überlebenszeiten gegenüber Infektionskontrolle in % | | Dosis-verhältnis |
|---|---|---|---|
| 2,5 / 5 / 2,5/5 | +50,0* | | 1 : 2 |
| 2,5 / 2,5 / 2,5/2,5 | +65,4* | | 1 : 1 |
| 1,875 / 5 / 1,875/5 | +66,7* | | 1 : 2,67 |
| 1,875 / 2,5 / 1,875/2,5 | +66,7* | | 1 : 1,33 |
| 1,25 / 20 / 1,25/20 | +55,2* | | 1 : 16 |
| 1,25 / 10 / 1,25/10 | +26,9* | | 1 : 8 |
| 1,25 / 7,5 / 1,25/7,5 | +74,3* | | 1 : 6 |
| 1,25 / 5 / 1,25/5 | +70,5* | | 1 : 4 |
| 0,625 / 20 / 0,625/20 | +47,4* | | 1 : 32 |
| 0,625 / 10 / 0,625/10 | +26,9* | | 1 : 16 |

FIG. 8

| DOSIS mg/kg (5x) | Verlängerte Überlebenszeiten gegenüber Infektionskontrolle in % | Dosis-verhältnis |
|---|---|---|
| 2,5 / 25 / 2,5/25 | + 26,1 * | 1 : 10 |
| 2,5 / 12,5 / 2,5/12,5 | +21,8 * | 1 : 5 |
| 1,25 / 100 / 1,25/100 | +114,4 * | 1 : 80 |
| 1,25 / 50 / 1,25/50 | +131,8 * | 1 : 40 |
| 1,25 / 25 / 1,25/25 | +143,4 * | 1 : 20 |
| 1,25 / 12,5 / 1,25/12,5 | +139,1 * | 1 : 10 |
| 1,25 / 6,25 / 1,25/6,25 | +57,9 * | 1 : 5 |

FIG. 9